# EUROPEAN PATENT APPLICATION

(11) **EP 2 450 002 A1**
(43) Date of publication of application: **09.05.2012**
(21) Application number: 09846825.9
(22) Date of filing: 03.07.2009
(51) Int. Cl.: A61C 8/00, A61B 18/00

(54) **ULTRASONIC TREATMENT APPARATUS**

(71) Applicant: ITO Co., Ltd., Bunkyo-ku Tokyo 113-0001 (JP); Bio Map Co., Ltd., Mizuho-shi Gifu 501-0213 (JP)
(72) Inventor: OHTA, Atsumi, Tokyo 176-0014 (JP); KAJIMOTO, Naoko, Mizuho-shi Gifu 501-0213 (JP); KAJIMOTO, Kyosuke, Mizuho-shi Gifu 501-0213 (JP)
(74) Representative: Derry, Paul Stefan
(86) International application number: PCT/JP2009/062203
(87) International publication number: WO 2011/001532

(57) **Abstract**

An ultrasonic therapy apparatus having an oscillator (2) connected to an electric power supplying unit through a cord (2b) and an elongated apparatus main body (3) through which the cord (2b) passes in the longitudinal direction and holding the oscillator (2) at a front end portion of the apparatus main body, in which the apparatus main body (3) includes a base member (10) having a holding unit (11) that holds the oscillator (2) and an accommodating groove (12) that accommodates the cord (2b), and a cover member (20) that covers the accommodating groove (12), the base member (10) and the cover member (20) are separably locked by a front end locking portion (31) provided in front of the front end portion and a base end locking portion (33) provided at a base end portion of the apparatus main body.

## Description

### Technical Field

The present invention relates to an ultrasonic therapy apparatus that is used to accelerate healing of a diseased portion in which an artificial dental root (implant) is placed.

### Background Art

Conventionally, a medical treatment has been utilized in which a pin-shaped artificial dental root called an "implant" is placed in the upper jaw or lower jaw, and an artificial crown is fitted onto the implant by a screw. A certain period of time is required for the implant to settle in the structure of a jaw bone of a patient, but the settlement period can be shortened by irradiating ultrasonic waves to the diseased portion in which the implant is placed.

For example, the ultrasonic therapy apparatus disclosed in the following Patent Document 1 has an oscillator connected to an electric power supplying unit through a cord and an elongated apparatus main body through which the cord passes in the longitudinal direction and a holding unit formed at the front end portion to hold the oscillator. The apparatus main body is gripped in the hand, the oscillator is placed in the vicinity of a diseased portion in the mouth, and ultrasonic waves generated by the oscillator are irradiated to the diseased portion.

### Citation List

### Patent Document

[Patent Document 1] Specification of Korean Patent No. 10-0844491

### Summary of Invention

### Technical Problem

However, the oscillator and the apparatus main body are integrally formed in the related art, and therefore there are problems in that it is difficult to clean and sterilize the inside of the apparatus main body or the portion between the oscillator and the apparatus main body, and a lot of time and effort is wasted or a large-scale apparatus needs to be used for the cleaning and sterilization operations. For example, when the ultrasonic therapy apparatus is used in a medical facility, it is necessary to frequently carry out cleaning and sterilization operations, and therefore a large-scale apparatus is necessary. Meanwhile, when the ultrasonic therapy apparatus is used at home, it is difficult to use a large-scale apparatus in practice, and therefore a lot of time and effort is unavoidably wasted.

In addition, when the oscillator is used a predetermined number of times, the oscillator may no longer satisfy the performance level appropriate for medical care, and thus there is a problem in the related art in that the apparatus main body also needs to be disposed of due to the performance degradation of the oscillator, which is uneconomic. Similarly, there is another problem in that the oscillator and the apparatus main body are not easily separately disposed, and are not easily recycled.

The invention has been made in consideration of the above circumstances, and the objects of the invention are as follows.
(1) To facilitate the cleaning and sterilization operations of the ultrasonic therapy apparatus
(2) To improve the economic efficiency of the ultrasonic therapy apparatus
(3) To facilitate the separate disposal of the oscillator and the apparatus main body, and improve the recycling properties

### Solution to Problem

In order to achieve the objects, the invention employs the following apparatus.

The invention has an oscillator connected to an electric power supplying unit through a cord and an elongated apparatus main body through which the cord passes in the longitudinal direction and holding the oscillator at a front end portion of the apparatus main body, in which the apparatus main body includes a base member having a holding unit that holds the oscillator and an accommodating groove that accommodates the cord, and a cover member that covers the accommodating groove, the base member and the cover member are separably locked by a front end locking portion provided in front of the front end portion and a base end locking portion provided at a base end portion of the apparatus main body.

According to this configuration, since the apparatus main body is composed of the base member and the cover member, and the base member and the cover member are separably locked by the front end locking portion and the base end locking portion, the base member and the cover member can be separated by releasing the locking of the front end locking portion and the base end locking portion. Thereby, the base member and the cover member are separated so that the inside of the apparatus main body or the portion between the oscillator and the apparatus main body can be cleaned and sterilized, and these operations can be facilitated.

In addition, since the base member and the cover member are separably locked, even when the oscillator no longer satisfies the performance level appropriate for medical care due to being used a predetermined number of times, the base member and the cover member are separated so that only the oscillator can be exchanged. Thereby, the apparatus main body can be reused, and the economic efficiency of the ultrasonic therapy apparatus can be improved.

Furthermore, since the base member and the cover member are separably locked, it is easy to separately dispose of the oscillator and the apparatus main body, and the recycling properties can be improved.

In addition, the ultrasonic therapy apparatus is preferably configured to have a front end locking wall protruding outward from the side wall of the accommodating groove provided at the front end side of the base member and a front end locking claw protruding inward from the side portion at the front end side of the cover member. Accordingly, the front end locking wall and the front end locking claw engage at the front end locking portion so as to prevent the base member and the cover member from being separated from each other.

In addition, it is preferable to provide a guiding groove that guides the front end locking claw to the engaging location between the front end locking claw with the front end locking wall at the side portion of the base member.

In addition, the length of the opening of the guiding groove is preferably formed to be larger than the thickness of the front end locking claw.

In addition, the ultrasonic therapy apparatus is preferably configured to have a base end dent portion provided at the side wall of the accommodating groove on the base end side of the base member and a base end locking claw protruding outward from the side portion provided on the base end side of the cover member so that the dent portion and the base end locking claw engage at the base end locking portion so as to prevent the base member and the cover member from being separated from each other.

In addition, it is preferable to provide an infeed penetrating in the thickness direction and extending in the longitudinal direction in the cover member.

In addition, it is preferable to have an intermediate locking portion that separably locks the base member and the cover member between the front end locking portion and the base end locking portion.

### Advantageous Effects of Invention

According to the ultrasonic therapy apparatus of the invention, it is possible to facilitate the cleaning and sterilization operation of the ultrasonic therapy apparatus. In addition, it is possible to improve the economic efficiency of the ultrasonic therapy apparatus, facilitate the separate disposal of the oscillator and the apparatus main body, and improve the recycling properties.

### Brief Description of Drawings

FIG. 1 is a perspective view of the appearance configuration of the ultrasonic therapy apparatus 1 according to an embodiment of the invention.
FIG. 2 is an exploded perspective view of the ultrasonic therapy apparatus 1 according to an embodiment of the invention.
FIG. 3 is a partial enlarged side view of the apparatus main body 3 in the ultrasonic therapy apparatus 1 according to an embodiment of the invention.
FIG. 4 is an explanatory view of a first action of the ultrasonic therapy apparatus 1 according to an embodiment of the invention, showing a process of combining the oscillator 2 with the base member 10.
FIG. 5 is an explanatory view of a second action of the ultrasonic therapy apparatus 1 according to an embodiment of the invention, showing a process of locking the front ends of the base member 10 and the cover member 20.
FIG. 6 is a partial enlarged side view of the second action of the ultrasonic therapy apparatus 1 according to an embodiment of the invention, showing an action.
FIG. 7 is a partial enlarged side view of the second action of the ultrasonic therapy apparatus 1 according to an embodiment of the invention, showing another action different from the action in FIG. 6.
FIG. 8 is an explanatory view of a third action of the ultrasonic therapy apparatus 1 according to an embodiment of the invention, in which the middle part of the ultrasonic base member 10 and the base end portion of the cover member 20 are locked so as to complete the assembly of the ultrasonic therapy apparatus 1.
FIG. 9 is an explanatory view of a fourth action of the ultrasonic therapy apparatus 1 according to an embodiment of the invention, in which the locking of the base end portion 3d of the apparatus main body 3 is released.
FIG. 10 is an explanatory view of a fifth action of the ultrasonic therapy apparatus 1 according to an embodiment of the invention, in which the locking of the front end portion 3a and middle portion of the apparatus main body 3 is released.

### Description of Embodiments

Hereinafter, embodiments of the invention will be described with reference to the drawings.

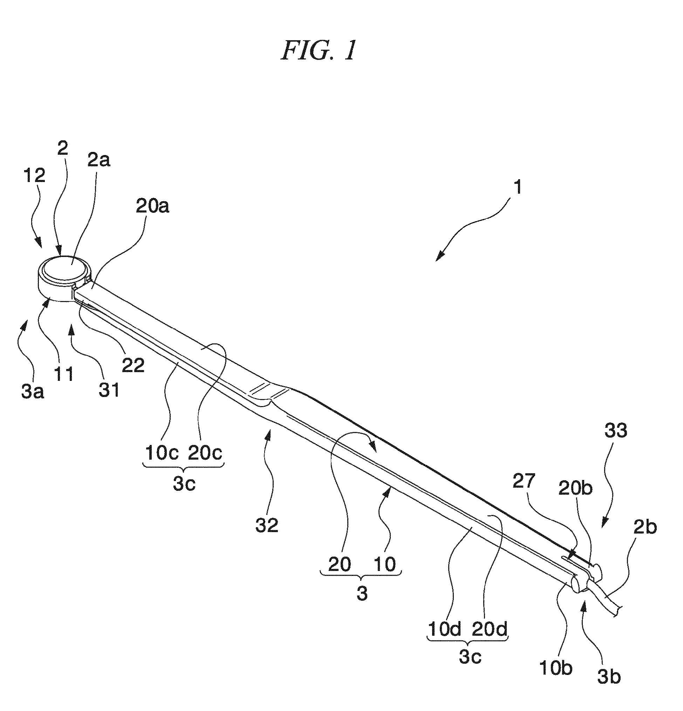

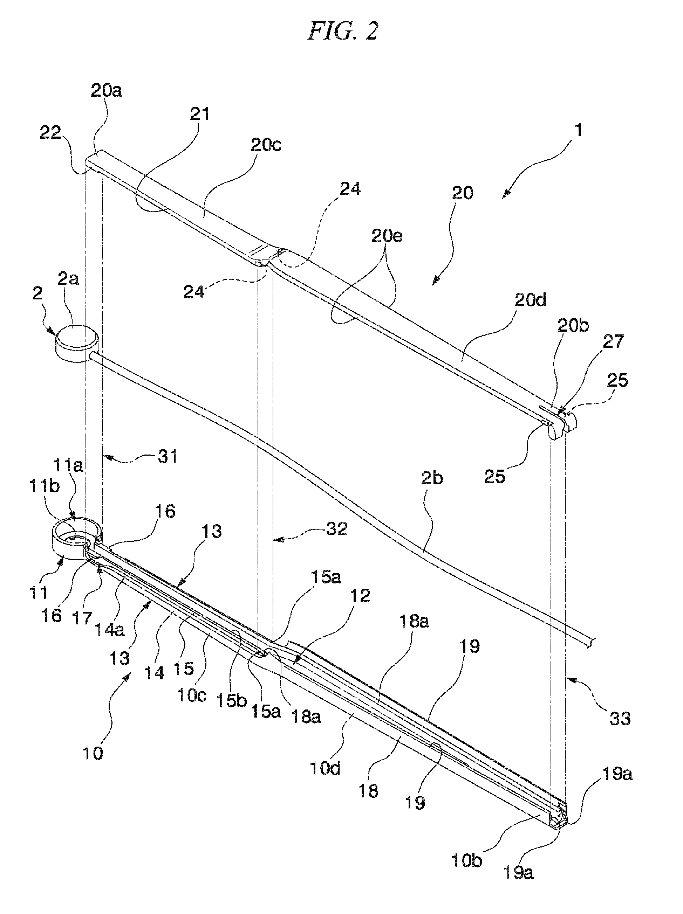

FIG. 1 is a perspective view of the appearance configuration of the ultrasonic therapy apparatus 1 according to an embodiment of the invention, and FIG. 2 is an exploded perspective view of the ultrasonic therapy apparatus 1.

As shown in FIG. 1, the ultrasonic therapy apparatus 1 has an oscillator 2 that irradiates ultrasonic waves onto a diseased portion and an apparatus main body 3 having a gripping function.

The oscillator 2 is made of a disc-shaped piezoelectric element and covered with aluminum or stainless steel in a state in which an irradiating circle face 2a is exposed as shown in FIG. 1. A cord 2b that can be connected to an external frequency inputting device (electric power supplying unit) is connected to the oscillator 2.

As shown in FIG. 1, the apparatus main body 3 is an elongated unit, holds the oscillator 2 at the front end portion 3a, and has a relatively thin and elastic head portion 3c and a gripping portion 3d that is configured to be thicker than the head portion 3c so that a user can easily grip the apparatus main body, which sequentially extend toward a base end portion 3b from the front end portion 3a.

The apparatus main body 3 is composed of a base member 10 and a cover member 20.

The base member 10 is an elongated resin member and has a holding portion 11 that holds the oscillator 2 at the front end as shown in FIG. 2. In addition, a base head portion 10c that composes a part of the head portion 3c and a base gripping portion 10d that is configured to be larger in the thickness dimension and the width dimension than the base head portion 10c sequentially extend toward the base end 10b from the holding portion 11.

The base head portion 10c and the base gripping portion 10are formed into a U shape in the cross section, and an accommodating groove 12 continues the extension in the longitudinal direction of the base member 10.

As shown in FIG. 2, the holding portion 11 is formed into a frame shape, and has an accommodating hole 11a that fits and accommodates the oscillator 2 in a state in which the irradiation circle face 2a is exposed. The accommodating hole 11a communicates with the outside through a hole 11b formed at the bottom portion and communicates with the accommodating groove 12 on the base end 10b side of the accommodating hole 11a.

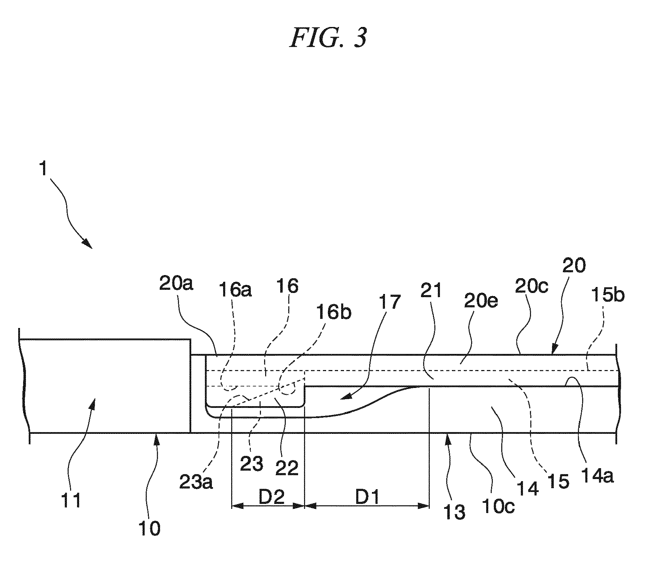

FIG. 3 is a partial enlarged side view of the apparatus main body 3.

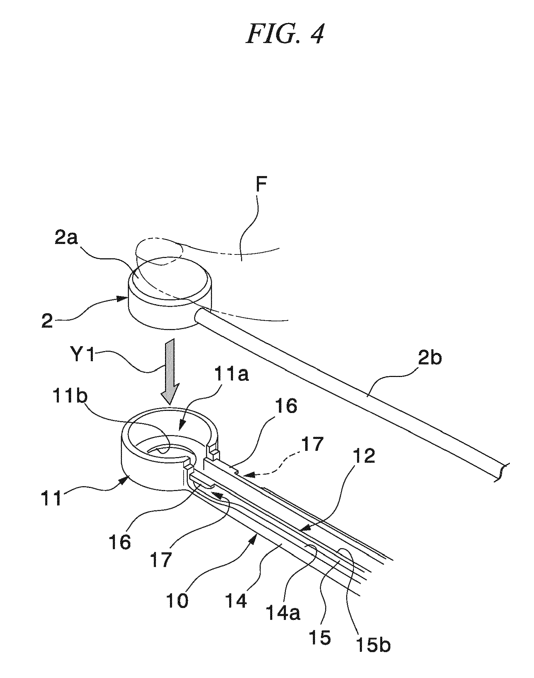

As shown in FIGS. 2 and 3, each of both side walls 13 at the base head portion 10c has a main wall 14, a thin inner wall 15, a front end locking wall 16, and a guiding groove 17 (refer to FIGS. 4, 6, and 7 if necessary).

The main wall 14 is continuously formed from the bottom portion of the base head portion 10c and configured to be relatively thick (refer to FIG. 4).

The thin inner wall 15 is shaped to protrude in the groove depth direction of the accommodating groove 12 (hereinafter referred to simply as the "groove depth direction") along the accommodating groove 12 on the inward side of the end face 14a of the main wall 14. In other words, the main wall 14 and the thin inner wall 15 form a step shape in the groove width direction of the accommodating groove 12 (hereinafter referred to simply as the "groove width direction").

As shown in FIG. 2, the thin inner wall 15 extends from the base gripping portion 10d to the front end locking wall 16, and an engaging protrusion portion 15a protruding in the normal direction (the groove width direction) of the thin inner wall 15 is formed on the base end 10b side of the thin inner wall 5.

As shown in FIGS. 2 and 4, the front end locking wall 16 is a plate-like portion protruding outward in the normal direction (groove width direction) of the thin inner wall 15, and extends in the longitudinal direction of the base head portion 10c so as to be adjacent to the holding portion 11 A chamfered portion 16b is formed on the base end 10b side of the lower face 16a of the front end locking wall 16 as shown in FIG. 3.

As shown in FIGS. 2 to 4, the guiding groove 17 is a thin portion continuously formed from the thin inner wall 15, has a dimension gradually increased in the groove depth direction of the accommodating groove 12 and then kept substantially constant, and extends toward the bottom of the front end locking wall 16. That is, the end face 14a of the main wall 14 is configured to extend toward the groove bottom side of the accommodating groove 12 in a smooth arc shape and then, again, extend in parallel to the front end locking wall 16 in a smooth arc shape.

The dimension of the guiding groove 17 at the portion continued from the thin inner wall 15, in other words, the length of the opening of the base head portion 10c in the longitudinal direction is D₁.

As shown in FIG. 2, the accommodating groove 12 in the base gripping portion 10d is configured to be larger than the groove depth and the groove width of the base head portion 10c and a main wall 18 and a thin outer wall 19 are formed at the side wall 13.

As shown in FIG. 2, the main wall 18 is a portion continuously formed from the bottom portion of the base head portion 10c, and formed continuously from the main wall 14 in the longitudinal direction of the base member 10. The end face 18a of the main wall 18 is connected with the end face 10b of the thin inner wall 15.

As shown in FIG. 2, the thin outer wall 19 is formed on the outward side of the end face 18a of the main wall 18 so as to protrude in the groove depth direction along the accommodating groove 12, and extends from the base head portion 10c to the base end 10b.

A base end dent portion 19a dented outward is shaped on the inner wall face on the base end 10b side of the thin outer wall 19.

As shown in FIG. 2, the cover member 20 is a resin member shaped into an elongated thin plate shape, configured to be slightly shorter than the base member 10, and covers the accommodating groove 12. The cover member 20 has a cover head portion 20c that composes a part of the head portion 3c and a cover gripping portion 20d having a larger thickness than the cover head portion 20c, which sequentially extend toward the base end 20b from the front end 20a.

As shown in FIGS. 2 and 3, protruding walls 21 protruding toward the groove bottom side of the accommodating groove 12 extend in the longitudinal direction of the cover member 20 at both side portions 20e of the cover member 20.

The length in the groove width direction between the protruding walls 21 in the cover head portion 20c is substantially the same as the length between the outer wall faces of the thin inner walls 15 at both side walls 13.

In addition, as shown in FIG. 3, a front end protrusion portion 22 protruding toward the groove bottom side more than the protrusion wall 21 is provided at both side portions 20e on the front end 20a side of the cover head portion 20c, and a front end locking claw 23 protrudes toward the inward side from the end portion of the front end protrusion portion 22. In the front end locking claw 23, an inner face intersecting in the thickness direction forms a slanted portion 23a that is located gradually closer to the groove bottom side of the accommodating groove 12 from the base end 20b side to the front end 20a side.

The front end protrusion portion 22 has a thickness (the length of the cover member 20 in the longitudinal direction) of D2 that is configured to be smaller than the length of the opening D1 of the guiding groove 17 as shown in FIG. 3.

An engaging dent portion 24 dented outward in the groove width direction is formed on the inner wall face of the protrusion wall 21 on the base end 20b side in the cover head portion 20c.

The length of the protrusion wall 21 in the groove width direction in the cover gripping portion 20d is substantially the same as the length between the inner wall faces of the thin outer walls 19 in the side walls 13 of the base member 10. A base end locking claw 25 protruding outward in the normal direction (the groove width direction) is formed on the outer wall face of the base end 20b of the protrusion wall 21 in the cover gripping portion 20d.

In addition, an infeed 27 penetrating in the thickness direction of the cover member 20 (the groove depth direction) and extending in the longitudinal direction of the cover member 20 is formed at the base end 20b of the cover gripping portion 20d.

The base member 10 and the cover member 20 which are configured in the above manner are locked with each other by the front end locking portion 31, the intermediate locking portion 32, and the base end locking portion 33 so as to configure the apparatus main body 3.

That is, in the front end locking portion 31, the front end locking wall 16 and the front end locking claw 23 are overlapped in the groove depth direction, and the chamfered portion 16b and the slanted portion 23a are engaged so as to prevent the base member 10 and the cover member 20 from being separated from each other in the groove depth direction of the accommodating groove 12.

Similarly, in the intermediate locking portion 32, the engaging protrusion 15a and the engaging dent portion 24 are overlapped and engaged in the groove width direction of the accommodating groove 12 in a state in which the engaging protrusion 15a is located on the outward side of the accommodating groove 12 and the engaging dent portion 24 is located on the inward side of the accommodating groove 12 so as to prevent the base member 10 and the cover member 20 from being separated from each other.

Similarly, in the base end locking portion 33, the base end dent portion 19a and the base end locking claw 25 are overlapped and engaged in the groove width direction in a state in which the base end base end dent portion 19a is located on the outward side of the accommodating groove 12 and the base end locking claw 25 is located on the inward side of the accommodating groove 12 so as to prevent the base member 10 and the cover member 20 from being separated from each other.

Meanwhile, in this state, the thin inner wall 15 of the base head portion 10c is located inward and the protrusion wall 21 in the cover head portion 20c is located outward in the groove width direction so as to compose the head portion 3c. Similarly, the thin outer wall 19 of the base gripping portion 10d is located outward and the protrusion wall 21 in the cover gripping portion 20d is located inward in the groove width direction of the accommodating groove 12 so as to compose the gripping portion 3d.

Next, the action of the ultrasonic therapy apparatus 1, which is composed in the above manner, during cleaning will be described. Meanwhile, in the following description, a sequence of assembling the ultrasonic therapy apparatus 1 from a state of being disassembled (refer to FIG. 2) will be described.

Firstly, as shown in FIG. 4, the oscillator 2 is pushed into the accommodating hole 11a using a finger F in a state in which the extending directions of the accommodating groove 12 and the cord 2b are approximately overlapped so that the oscillator 2 is fitted into and accommodated in the holding portion 11 (arrow Y1).

After the oscillator 2 is accommodated in the holding portion 11 the cord 2b is accommodated in the accommodating groove 12 in the same manner as the oscillator 2.

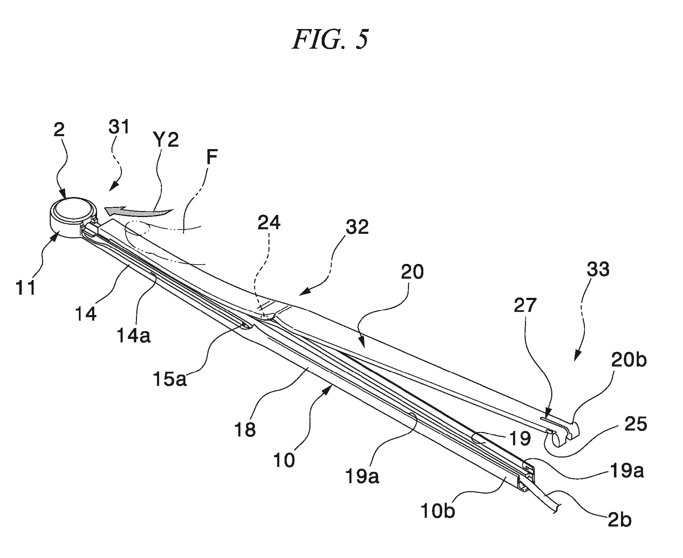

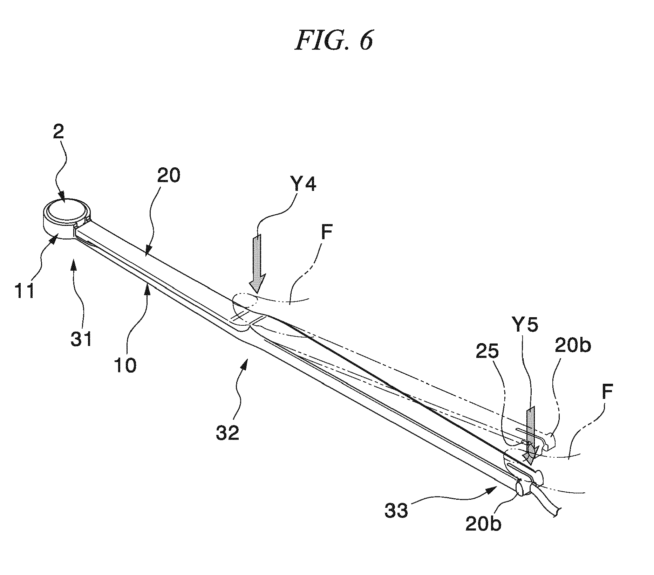

Next, as shown in FIGS. 5 and 6, the front end protrusion portion 22 is brought into contact with the end face 14a of the main wall 14, and the front end protrusion portion 22 is slid from the base end 10b side of the base member 10 to the holding portion 11side of the base member 10 (arrows Y2 and Y3).

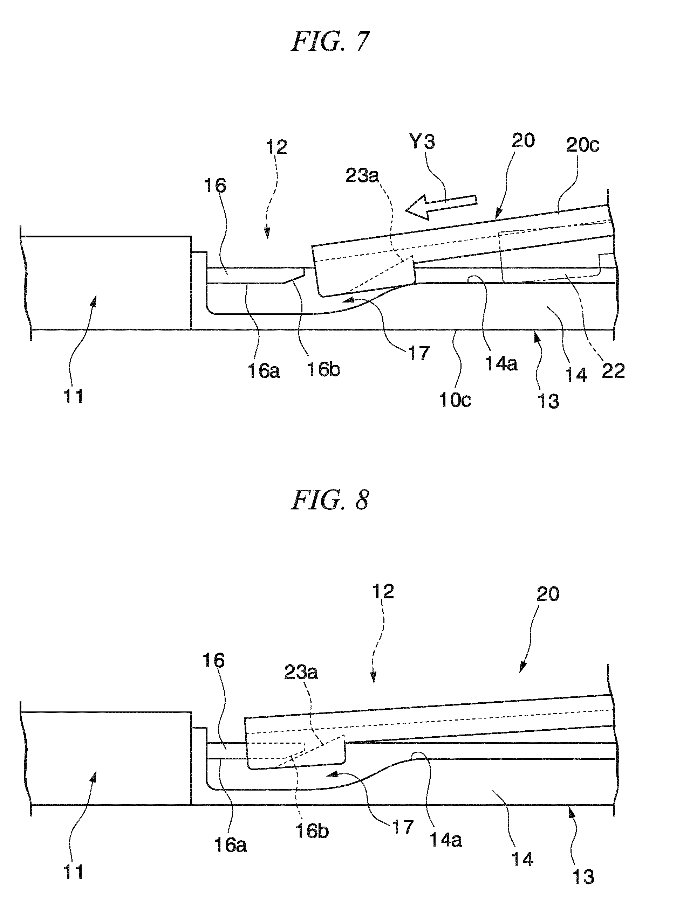

When the front end protrusion portion 22 that has slid on the end face 14a arrives at the guiding groove 17, the front end protrusion portion 22 is moved along the end face 14a toward the groove bottom side of the accommodating groove 12 as shown in FIG. 6. At this time, since the length of the opening D1 of the guiding groove 17 (refer to FIG. 3) is larger than the thickness D2 of the front end locking claw 23, the front end locking claw 23 is guided to the inside of the guiding groove 17 without being caught by the opening of the guiding groove 17. Furthermore, since the front end locking wall 16 has the chamfered portion 16b, and the front end locking claw 23 has the slanted portion 23a, the front end locking claw 23 is smoothly guided to the holding portion 11 side of the base member 10 and the groove bottom side of the accommodating groove 12 even when the front end protrusion portion 22 and the front end locking claw 23 comes into contact with each other as shown in FIG. 7.

The front end protrusion portion 22 is moved toward the holding portion 11 side of the base member 10 in the above manner as shown in FIG. 3, and the slanted portion 23a and the chamfered portion 16b are pressed, brought into contact, and locked with each other. That is, the front end locking portion 31 locks the front end sides of the base member 10 and the cover member 20.

After the front end sides of the base member 10 and the cover member 20 are locked as shown in FIG. 6, around the middle portion of the cover member 20 is pressed by the finger F in a state in which the base member 10 is supported so that the engaging protrusion portion 15a is fitted into the engaging dent portion 24 (arrow Y4). In the above manner, the engaging protrusion portion 15a and the engaging dent portion 24 are engaged, thereby locking the middle portions of the base member 10 and the cover member 20. At this time, the engaging dent portion 24 is located on the outward side of the groove width direction, and the engaging protrusion portion 15a is located on the inward side of the groove width direction, respectively.

Similarly, the base end 20b of the cover member 20 is pressed by the finger F in a state in which the base member 10 is supported so that the engaging protrusion portion 25 is fitted into the base end dent portion 19a as shown in FIG. 6. In the above manner, the base end locking claw 25 and the base end dent portion 19a are engaged, thereby locking the base ends of the base member 10 and the cover member 20. At this time, the base end dent portion 19a is located on the outward side of the groove width direction, and the base end locking claw 25 is located on the inward side of the groove width direction, respectively.

As described above, the base member 10 and the cover member 20 are locked at each of the front end locking portion 31, the intermediate locking portion 32, and the base end locking portion 33 as shown in FIG. 1 so that assembly of the ultrasonic therapy apparatus 1 is completed.

The ultrasonic therapy apparatus 1 has the cord 2b connected to an external frequency inputting device so as to input predetermined frequencies to the oscillator 2. The oscillator 2 to which the frequencies are input generates resonance and irradiates ultrasonic waves. That is, a user grips the gripping portion 3d, and places the oscillator 2 in the vicinity of a diseased portion in a mouth so that ultrasonic waves generated by the oscillator 2 are irradiated to the diseased portion.

Subsequently, a method of separating the ultrasonic therapy apparatus 1 will be described.

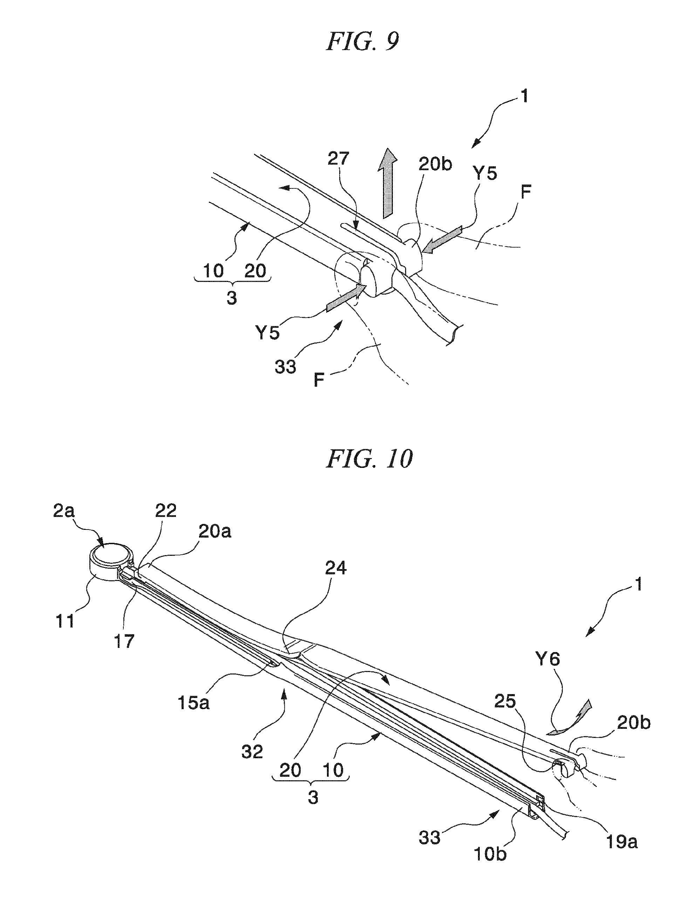

Firstly, the base end 20b of the cover member 20 is pressed inward in the groove width direction from both sides (arrow Y5) as shown in FIG. 9. When the base end 20b is pressed in, the base end 20b is elastically deformed so that the base end locking claw 25 and the base end dent portion 19a are separated, and the locking is released, whereby the locking of the base end locking portion 33 is released. In this manner, the locking of the base member 10 and the cover member 20 at the base ends is removed.

Next, the base end 10b and the base end 20b are separated in the groove depth direction as shown in FIG. 10 (arrow Y6).

As the separation of the base end 20b and the base end 10b progresses, the portion separating the base member 10 and the cover member 20 expands toward the front end 3a portion, and the locking of the engaging protrusion portion 15a and the engaging dent portion 24 is released. That is, the locking of the base end locking portion 33 is released, and the locking state of the base member 10 and the cover member 20 at the base ends is removed.

Similarly, the portion separating the base member 10 and the cover member 20 expands toward the front end portion 3a, the pressing friction between the chamfered portion 16b and the slanted portion 23a reaches the limit, the chamfered portion 16b and the slanted portion 23a slide in the face direction, and the engagement of the slanted portion 23a and the chamfered portion 16b is released (refer to FIGS. 6 and 7). After that, the base member 10 and the cover member 20 are separated by removing the front end protrusion portion 22 from the opening portion of the guiding groove 17 (refer to FIG. 2). After that, the oscillator 2 is removed by inserting the finger F or the like into the hole 11b so that the ultrasonic therapy apparatus 1 is separated into the oscillator 2, the base member 10, and the cover member 20.

After the respective member separated in the above manner is cleaned and sterilized, the ultrasonic therapy apparatus 1 is assembled in the same manner as in the above, and ultrasonic wave medical treatment is repeatedly carried out without causing discomfort to patients.

As described above, according to the ultrasonic therapy apparatus 1 according to the present embodiment, since the apparatus main body 3 is composed of the base member 10 and the cover member 20, and the base member 10 and the cover member 20 are separably locked by the front end locking portion 31, the intermediate locking portion 32, and the base end locking portion 33, it is possible to separate the base member 10 and the cover member 20 by releasing the locking of the front end locking portion 31, the intermediate locking portion 32, and the base end locking portion 33. Thereby, it is possible to separate the base member 10 and the cover member 20, and carry out cleaning and sterilization operations of the inside of the apparatus main body 3 or the portion between the oscillator 2 and the apparatus main body 3, which facilitates these operations.

In addition, since the base member 10 and the cover member 20 are separably locked, even when the oscillator 2 no longer satisfies the performance level appropriate for medical care due to being used a predetermined number of times, it is possible to separate the base member 10 and the cover member 20 and exchange only the oscillator 2. Thereby, the apparatus main body 3 can be reused, and the economic efficiency of the ultrasonic therapy apparatus 1 can be improved.

Furthermore, since the base member 10 and the cover member 20 are separably locked, it is easy to separately dispose of the oscillator 2 and the apparatus main body 3, and it is possible to provide the ultrasonic therapy apparatus 1 having improved recycling properties.

In addition, the front end locking wall 16 protruding outward from the side wall 13 of the base member 10 and the front end locking claw 23 protruding inward from the protrusion wall 21 of the side portion 20e on the front end 20a side of the cover member 20 are provided. Accordingly, the front end locking wall 16 and the front end locking claw 23 engage at the front end locking portion 31 so as to prevent the base member 10 and the cover member 20 from being separated from each other. Accordingly, it is possible to firmly lock the front end portion 3a of the apparatus main body 3 with a simple configuration, and easily release the locking.

In addition, since the guiding groove 17 that guides the front end locking claw 23 to the engaging location between the front end locking claw 23 with the front end locking wall 16 is provided, it is possible to easily carry out the assembly, and user can appropriately assemble the ultrasonic therapy apparatus 1 particularly when carrying out the assembling at home.

In addition, since the length of the opening D₁ of the guiding groove 17 is formed to be larger than the thickness D₂ of the front end locking claw 23, it is possible to facilitate introduction of the front end locking claw 23 to the guiding groove 17. Thereby, it is possible to more easily carry out the assembly of the ultrasonic therapy apparatus 1.

In addition, since the base end dent portion 19a is provided at the side wall 13 of the accommodating groove 12 of the base member 10, and the base end locking claw 25 protruding outward from the protrusion wall 21 at the side portion is provided on the base end 20b side of the cover member 20. Accordingly, the base end dent portion 19a and the base end locking claw 25 engage at the base end locking portion 33 so as to prevent the base member 10 and the cover member 20 from being separated from each other. Therefore, it is possible to firmly lock the base end portion 3b of the apparatus main body 3 with a simple configuration, and easily release the locking.

In addition, since the infeed 27 penetrating in the thickness direction and extending in the longitudinal direction is provided in the cover member 20, it is possible to elastically deform the base end 20b so as to easily release the locking of the base end locking portion 33.

In addition, since the ultrasonic therapy apparatus 1 has the intermediate locking portion 32 that separably locks the base member 10 and the cover member 20 between the front end locking portion 31 and the base end locking portion 33, it is possible to further firmly lock the base member 10 and the cover member 20. Particularly, even when the oscillator 2 is pressed on a diseased portion, it is possible to sufficiently suppress the head portion 3c and the gripping portion 3d from being separated.

Meanwhile, the action sequence, the shapes, combinations, and the like of the respective constituent members in the above embodiment are simply examples, and a variety of modifications are permitted based on design requirements and the like within a scope not departing from the gist of the invention.

For example, in the embodiment, the holding portion 11 is configured to hold the oscillator 2 so as to fit and accommodate the oscillator 2, but the oscillator 2 may be held by other methods, for example, pinning or using other members.

In addition, the embodiment is configured to provide the intermediate locking portion 32, but the intermediate locking portion 32 is not necessarily provided, and it is still possible to sufficiently lock the base member 10 and the cover member 20 even if only the front end locking portion 31 and the base end locking portion 33 are provided.

In addition, the embodiment is configured to have the chamfered portion 16b provided at the front end locking wall 16, but the chamfer portion 16b is not necessarily provided. For example, as long as the length of the opening D1 is configured to be larger than the thickness D2 of the front end locking claw 23 as in the embodiment, it is possible to rotate and screw in the front end locking claw 23. In addition, the front end locking claw 23 may be locked simply by pressing in.

### Reference Signs List

- 1: ULTRASONIC THERAPY APPARATUS
- 2: OSCILLATOR
- 3: APPARATUS MAIN BODY
- 3a: FRONT END PORTION
- 3b: BASE END PORTION
- 10: BASE MEMBER
- 10a: FRONT END OF THE BASE MEMBER
- 10b: BASE END OF THE BASE MEMBER
- 11: HOLDING PORTION
- 12: ACCOMMODATING GROOVE
- 13: SIDE WALL
- 16: FRONT END LOCKING WALL
- 17: GUIDING GROOVE
- 20: COVER MEMBER
- 20a: FRONT END OF THE COVER MEMBER
- 20b: BASE END OF THE COVER MEMBER
- 20e: SIDE PORTION
- 22: FRONT END PROTRUSION PORTION
- 23: FRONT END LOCKING CLAW
- 25: BASE END LOCKING CLAW
- 27: INFEED
- 31: FRONT END LOCKING PORTION
- 32: INTERMEDIATE LOCKING PORTION
- 33: BASE END LOCKING PORTION
- D1: LENGTH OF OPENING
- D2: THICKNESS

## Claims

1. An ultrasonic therapy apparatus, comprising an oscillator connected to an electric power supplying unit through a cord and an elongated apparatus main body through which the cord passes in the longitudinal direction and holding the oscillator at a front end portion of the apparatus main body,
wherein the apparatus main body includes a base member having a holding unit that holds the oscillator and an accommodating groove that accommodates the cord, and a cover member that covers the accommodating groove,
the base member and the cover member are separably locked by a front end locking portion provided in front of the front end portion and a base end locking portion provided at a base end portion of the apparatus main body.

2. The ultrasonic therapy apparatus according to Claim 1,
wherein a front end locking wall protruding outward from the side wall of the accommodating groove is provided at the front end side of the base member,
a front end locking claw protruding inward from the side portion at the front end side of the cover member is provided, and
the front end locking wall and the front end locking claw engage at the front end locking portion so as to prevent the base member and the cover member from being separated from each other.

3. The ultrasonic therapy apparatus according to Claim 1 or 2,
wherein a guiding groove that guides the front end locking claw to an engaging location between the front end locking claw with the front end locking wall is provided at the side portion of the base member.

4. The ultrasonic therapy apparatus according to any one of Claims 1 to 3,
wherein the length of the opening of the guiding groove is formed to be larger than the thickness of the front end locking claw.

5. The ultrasonic therapy apparatus according to any one of Claims 1 to 4,
wherein a base end dent portion is provided at the side wall of the accommodating groove on the base end side of the base member,
a base end locking claw protruding outward from the side portion is provided on the base end side of the cover member, and
the dent portion and the base end locking claw engage at the base end locking portion so as to prevent the base member and the cover member from being separated from each other.

6. The ultrasonic therapy apparatus according to any one of Claims 1 to 5,
wherein an infeed penetrating in the thickness direction and extending in the longitudinal direction is provided in the cover member.

7. The ultrasonic therapy apparatus according to any one of Claims 1 to 6, further comprising an intermediate locking portion that separably locks the base member and the cover member provided between the front end locking portion and the base end locking portion.
